# EUROPEAN PATENT SPECIFICATION

(11) **EP 2 005 131 B1**
(45) Date of publication and mention of the grant of the patent: **18.11.2009**
(21) Application number: 07736168.1
(22) Date of filing: 01.04.2007
(51) Int. Cl.: G01K 13/00, A61B 5/00

(54) **IMPROVED DIGITAL THERMOMETER**
VERBESSERTES DIGITALTHERMOMETER
THERMOMÈTRE NUMÉRIQUE AMÉLIORÉ

(30) Priority: 03.04.2006 IL 17473606; 17.10.2006 IL 17867306
(43) Date of publication of application: 24.12.2008
(73) Proprietor: HOW-R-YOU TECHNOLOGIES LTD., 92261 Jerusalem (IL)
(72) Inventor: MENASHE, Ilan, Ashdod 77452 (IL)
(74) Representative: Petruzziello, Aldo
(86) International application number: PCT/IL2007/000428
(87) International publication number: WO 2007/113825

(56) References cited:
- WO-A-2005/092177

## Description

### FIELD OF THE INVENTION

The invention relates to a clinical thermometer and use thereof.

### BACKGROUND OF THE INVENTION

Many clinical thermometers in use today are built for accomplishing fast, precise body temperature measurements. The typical construction of such thermometers includes a plastic, waterproof case housing a temperature sensor, electronic circuitry and a digital display. The electronic circuitry is designed to receive a temperature signal from the temperature sensor, translate the signal into a value representative of the measured temperature, and display the value on the digital display.

US Pat. No. 5,056,048 to Seperant, discloses such a thermometer for precision measurement of body temperature wherein an analog signal corresponding to resistance of a temperature sensing element is converted into a digital signal. The functionality of the converter may be realized by a microcomputer, in order to effect precision measurements, which are stated to reach a precision level of 0.001 degree Celsius.

US Pat. No. 5,295,746 to Friauf et al. discloses a thermometer capable of measuring temperature differences in the order of several micro-degrees centigrade.

US Pat. No. 6,656,115 (Miyazaki et al.) discloses a medical information system wherein sensors are provided for measuring information used as indices of the condition of a patient's health. A database stores patient data such as temperature, pulse, fat, weight, blood pressure, blood sugar, and so on. Also stored for each measured value are corresponding thresholds for each patient. For example, a patient A may be abnormal if his or her temperature exceeds 37.0°, while a patient B may be normal until his or her temperature exceeds 38.0°. Measurements are taken based on a stored schedule for each patient and may be conveyed automatically to a health administrator if the value of the measured parameter is not within the predetermined range of values.

US Pat. No. 5,626,425 (Fujikawa et al.) discloses an electronic thermometer for measuring body temperature having a temperature sensor for detecting a sensed temperature and for producing an output indicative of the sensed temperature and a temperature rise detector for detecting whether the sensed temperature is rising.

WO 01/33178 discloses a clinical thermometer which includes a visual indication of the temperature of the patient relative to normal body temperature. An alternative thermometer is also disclosed in which an alarm is triggered if the temperature of the patient rises (or falls) too rapidly. In a still further arrangement, a trend indicator is provided to represent changes in the measured temperature over time.

There is also a vast literature relating to steady-state temperature prediction based on an instantaneous measurement so as thereby to reduce the time interval for taking the body temperature. For example, US Pat. No. 4,878,184 (Okada et al.) and assigned to Omron Tateisi Electronics Co. discloses an electronic thermometer comprising a temperature sensor, and a memory for storing a measured value T(t) representing the instantaneous temperature. A prediction unit computes a predicted value S(t) based on the measured value T(t), and a display device displays the measured or the predicted value.

Likewise, US Pat. 4,986,669 (Yamaguchi) and assigned to Terumo Corporation discloses an electronic clinical thermometer for predicting a sensed temperature that will prevail at a future time by applying a predictive functional formula specified by coefficient parameters, which define an estimated shape of a temperature rise curve of a temperature probe when body temperature is measured.

Also well-known in the art are temperature-based devices for predicting the date of ovulation. For example, JP 62238428 describes a device that stores temperatures measured at predetermined times and forecasts from the measured temperatures a coming physiological state for a predetermined period for subsequent display. In this and similar devices, the woman's temperature is measured daily at the same time of day throughout her menstrual cycle and the results are stored for evaluating an increase in temperature that is indicative of ovulation. Such prediction cannot be made on a series of temperature samples obtained in a single measurement.

US 2004/254472 (McQuilkin) assigned to Cardiowave Inc. proposes the use of thermal imaging to detect core body temperature in a patient noninvasively, remotely and accurately allowing rapid screening for diseases or conditions that are characterized by changes in core body temperature. Suggested diseases or conditions that can be screened include severe acute respiratory syndrome (SARS), since fever is a common, early symptom. It is also speculated that a similar approach may be used to detect other infectious diseases and conditions induced by biological weapons by an increase in body temperature. Conversely, hypothermia from exposure or surgery may be detected by a decrease in core body temperature. Core body temperature is computed using a heat transfer model from known surface skin temperature and ambient temperatures, provided by thermal imaging.

In similar vein, WO2005092177 discloses a non-invasive temperature monitoring device for detecting human physiological or contextual information. The device is adapted to measure temperature fluctuations within an accuracy of better than 0.177°C so as to determine a departure from baseline body temperature of 2-3°C. Device accuracy is predicated on the assumption that clinical or medical applications require an accuracy level having a mean error of better than 0.5 degrees Celsius.

The contents of all the above-mentioned references are fully incorporated herein by reference.

To summarize, the prior art describes thermometers able to resolve temperatures to within 0.01°C. It also describes techniques for predicting specific body changes such as ovulation based on repeated temperature measurements taken over a protracted period of time. It likewise teaches accurate temperature measurement from a few successive samples allowing prediction based on a measured rate of change of the samples. Finally, accurate derivation of core body temperature has been suggested to screen various diseases or conditions.

However, it is often the case that an individual is overcome by bodily sensations associated with an abnormal body condition without exhibiting a significant body temperature change. In such instances the results from measuring instantaneous body temperature measurement may be misleading, as they would imply that the user is healthy, even though an abnormal body condition has already set in.

### SUMMARY OF THE INVENTION

It is an object of the present invention to provide a thermometer that will detect and alert a user to at least one health property other than temperature of the examinee or for predicting a change in at least one health property of the examinee.

In accordance with one aspect of the invention, there is provided an electronic thermometer, comprising a temperature measuring device for measuring temperature of an examinee, and a microprocessor coupled to the temperature measuring device for processing a sequence of temperature samples measured during a unitary measuring interval and being responsive to said temperature samples or a function thereof for determining at least one health property of the examinee or for predicting a change in at least one health property of the examinee;
**characterized in that:**
the temperature measuring device is adapted to measure to a resolution of at least 0.01 °C at a sufficiently high sampling rate to yield measurable fluctuations between a plurality of sampled data points within the measuring interval and to enable a health property to be determined or a change in the health property predicted from said fluctuations even when each measured temperature sample exhibits substantially no departure from normal body temperature.

The thermometer may be a standalone thermometer having an integral sensor, microprocessor and memory. Alternatively, it may include only a sensor and an output for coupling to an external computer.

At its most basic, the thermometer according to the invention allows the existence of an abnormal health condition to be identified even when not accompanied by an abnormally high or low temperature; and to accomplish this with a single temperature measurement over a short period allowing a number of digital samples to be obtained and analyzed, without the need to take repeated measurements over an extended period of time. In such case, the health property may simply be an indication that the patient is healthy or unhealthy. As noted above, thermometers are known which predict steady-state temperature based on an instantaneous measurement. However, such thermometers are unable to distinguish between a healthy patient and an unhealthy patient who shows no discernible departure from normal body temperature. As such, they are unable to identify an abnormal health condition that is not accompanied by an abnormally high or low temperature In contrast, the thermometer according to the invention allows determination of whether a patient is healthy or unhealthy even when each measured temperature sample exhibits no marked departure from normal body temperature. readings are taken. However, there appears to be no reason why the principles of the invention will not be applicable to other animals providing that temperature-dependent models can be established that allow accurate prediction of one or more identifiable health conditions.

In one aspect, the thermometer effects 25 or more body temperature readings per minute all relating to a unitary measurement sample. Within the context of the description and the appended claims, the term "unitary" is used to define a single continuous temperature sampling interval that may span over an extended period of time such as several minutes. The microprocessor may be programmed to reject readings that do not fulfill predetermined threshold criteria. One threshold criterion is the absolute value of a maximum slope of a straight line generated between first and last data points of a best-fit line; e.g. approximately 0.0009. Another threshold criterion is a maximum deviation between two adjacent data points. Yet another threshold criterion is a maximum deviation between the generated straight line and the one or more amplitudes of the best-fit line.

In one aspect, the microprocessor is operable to determine that an acceptable best-fit line fulfilling the predetermined threshold criteria indicates an abnormal body condition by means of an amplitude-based analysis.

The microprocessor is operable to match to compare measured temperature samples or a function thereof with predetermined data characteristics of one or more abnormal health conditions for predicting an onset of an abnormal health condition of the examinee. This has been done according to a first approximation by determining whether a generated best-fit line through the measured temperature samples sufficiently matches one of the stored patterns. According to another approach volatility of the temperature-time characteristic has been found to serve as a good indication of whether the examinee is healthy or not, even when no fever was registered. 'Volatility' is a measure of the extent to which temperature samples fluctuate with time, such as the number of temperature rises and falls during the sampling period.

According to some embodiments the thermometer includes an integral memory that stores a database of stored temperature fluctuation patterns or functions wherein each pattern or rule is characteristic of a different respective condition. In one embodiment that has been reduced to practice, the function normalizes the measured temperature samples by discarding uncharacteristic measurements that act to obscure statistically significant fluctuations and then analyses the remaining data to compute at least one statistically significant parameter that is indicative of a health condition. One such statistically significant parameter has been found to be volatility. If known temperature-time characteristics are known for different diseases or health conditions, the database may store the characteristics or functions thereof so as to allow measured temperature samples to be matched with a stored characteristic and thereby allow prediction of the onset of a specific disease or health condition. The database may also be customized to store respective sets of patterns each relating to a selected population sector. A selected population sector may include any cross-section of the general population such as males and females, as well as sub-groups thereof such as pregnant women, a pediatric population, a geriatric population, individuals suffering from heart conditions or other ailments. Sub-groups relating to different age ranges may also be used as well as combined groups, such as pregnant women over a given age. Sub-groups may also be confined to single individuals so as to allow the thermometer to be customized for use with a specific person. This allows different thermometers to be customized for use with different family members, for example, for each of whom normal baseline characteristics are stored.

The thermometer may include an output unit that may include a display device for displaying information indicative of a health condition of the examinee such as an icon or textual message indicative of a predicted condition. The display device may be adapted to display an instantaneous temperature of the user and a rate of temperature change thereof. The output unit may include a vocalization unit for vocalizing a suitable message, either as an adjunct to the display device or instead of it. Such a vocalization unit is of particular benefit to the visually impaired. The vocalization unit may serve to generate a beeping sound upon activation of the thermometer, and/or after a specified time interval such as 90 seconds following activation of the thermometer so as to indicate termination of the sampling period.

In one aspect the thermometer comprises a short range wireless transmitter for transmitting data that is received and generated by the microprocessor during the predetermined measuring time. The data that is received and generated by the microprocessor during the measuring time may be selected from the group of measuring time, stabilization time, a plurality of measured data points, the best-fit line, a deviation of the best-fit line from a stored pattern, the straight line, the slope of the straight line, a maximum deviation between two adjacent data points and deviation between one or more amplitudes of the best-fit line and the straight line.

In one embodiment, all components of the thermometer are housed in a single physical unit. Alternatively, the microprocessor may be disposed remotely to the temperature measuring device. Likewise, the database may be located remotely from the temperature measuring device.

The present invention may achieve accurate temperature measurement by employing simple, statistical methods for processing temperature readings. For example, a best-fit line, correlation coefficient and linear regression line, as well as the slope of the line may be calculated and used for detecting patient body condition as well as optionally providing accurate temperature indication. The temperature measuring device may be a conventional temperature probe having an accuracy of at least 0.01°C.

According to a first approximation that was found to produce statistically significant results with some data samples, the microprocessor may generate a line substantially coinciding with the shortest possible line between each pair of adjacent measurement data points. The generated line is smoothed, if required, to produce a single line having no abrupt changes in direction (referred to herein as a "best-fit line"). In accordance with some embodiments, the generated best-fit line, which may be linear or non-linear, is characteristic of a unique trend in a change in the body temperature of the examinee. The best-fit line is compared with the straight linear regression line generated between the first and last data points of said best-fit line to determine the body condition of said selected target. A best-fit line having a deviation less than a predetermined deviation from (hereinafter "basically similar to") a stored pattern or value is indicative that the examinee is undergoing a departure from a health condition corresponding to the stored pattern or value.

A best-fit line is not accepted by the microprocessor if it does not fulfill threshold criteria. After a best-fit line within a given measuring time is constructed, a straight line between first and last data points of the best-fit line is generated. When the slope of this straight line is greater than a predetermined value, the best-fit line associated with the generated straight line is indicative that the data points have been measured prior to the stabilization time and the best-fit line is therefore not accepted. The best-fit line is also not accepted when the minimum deviation between two ore more adjacent data points is greater than a predetermined correlation value, or correlation coefficient, indicating that background electrical noise has been detected.

Alternatively, as noted above, volatility of the temperature-time characteristic has been found to serve as a better indication of whether the examinee is healthy or not, even when no fever was registered. It will thus be realized that the novel device of the present invention serves to provide a good indication of an instantaneous health condition and can predict the health condition in the short-term future by detecting and analyzing instantaneous changes in body temperature.

While prior art digital thermometers are capable of determining body temperature to an accuracy of 0.01°C, they are adapted to determine the instantaneous body temperature, but not to process a plurality of samples of body temperature taken during a single reading so as to predict a characteristic temperature trend or a change in a baseline body condition. The digital thermometer of the present invention, in contrast, measures the body temperature, after a predetermined stabilization time, e.g. 10 seconds, at a sufficiently high sampling rate to achieve a plurality of measurement data points within a given short total measuring time, e.g. of 30 to 90 seconds, and at high resolution, e.g. of 0.01°C.

### BRIEF DESCRIPTION OF THE DRAWINGS

In order to understand the invention and to see how it may be carried out in practice, embodiments will now be described, by way of non-limiting example only, with reference to the accompanying drawings, in which:
**Fig. 1** is a block diagram showing functionality of the thermometer according to the invention;
**Fig. 2** is a pictorial representation of an integral thermometer according to an embodiment of the invention;
**Fig. 3** is a flow chart showing principal operations carried out by a processor in a thermometer according to an embodiment the invention;
**Figs. 4a** **and** **5a** are temperature-time characteristics of different healthy patients measured using the thermometer according to the invention;
**Figs. 4b** **and** **5b** show the normalized temperature-time characteristics of Figs. 4a and 5a;
**Figs. 6a** **and** **7a** are temperature-time characteristics of different unhealthy patients measured using the thermometer according to the invention; and
**Figs. 6b** **and** **7b** show the normalized temperature-time characteristics of Figs. 6a and 7a.

### DETAILED DESCRIPTION OF EMBODIMENTS

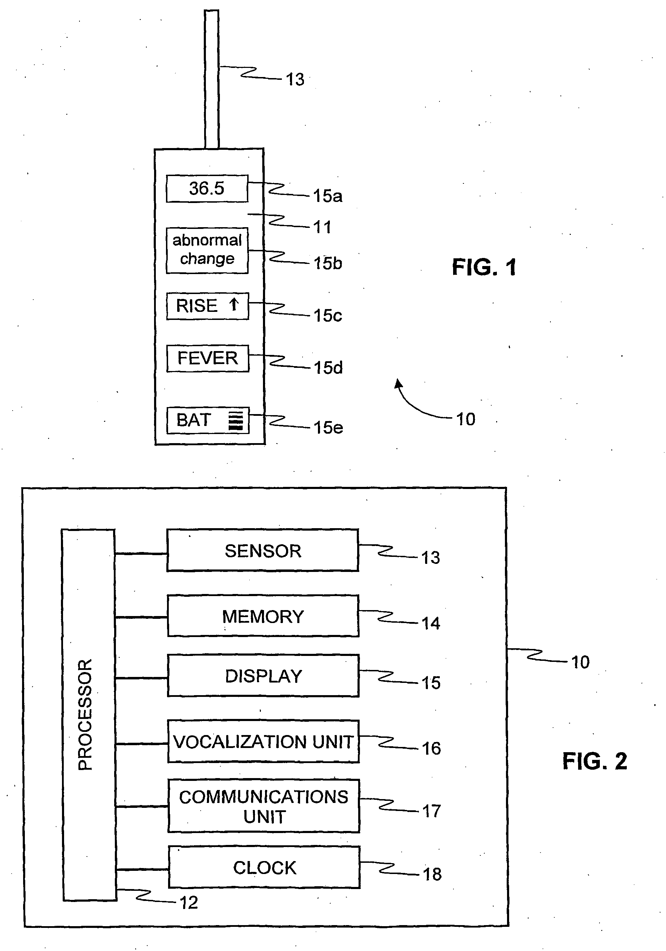

Referring to Figs. 1 and 2 there is shown functionally and pictorially an integral thermometer 10 according to an embodiment of the invention having a housing 11 accommodating a processor 12 to which there are coupled a temperature sensor 13 in the form of a probe protruding from the housing, a memory 14, a display 15, a vocalization unit 16 and a communications unit 17. The thermometer 10 is powered by a battery (not shown) that is accommodated within the housing. Timing signals are provided by a clock 18 coupled to the processor 12. The display 15 is adapted to display a suitable icon or textual message when the processor 12 predicts a change in a baseline status of the examinee. For example, a suitable icon or textual message may be displayed when the processor 12 predicts an onset of an abnormal health condition of the examinee. Preferably, the display 15 is adapted to display temperature and other data relating to a predicted change in a baseline status of the examinee. In one embodiment, the display 15 may comprise a unitary display device that is adapted to display these data sequentially, each for a predetermined time period such as several seconds, or in response to user selection via a suitable switch. Alternatively, separate display devices may be provided for the concurrent display of each of these data. This is shown in Fig. 1, where display devices 15a, 15b, 15c, 15d and 15e are provided to show, respectively, measured temperature in degrees Celsius, normal/abnormal change indication, temperature rise/fall indication, predicted health condition and battery status indication. The vocalization unit 16 vocalizes a suitable message, either as an adjunct to the display device or instead of it and produces an audible tone at the start and termination of a measurement sequence. The communications unit 17 allows for data communication between the thermometer and a remote device and may be constituted by a wireless transmitter, or by a wired interface such as RS232, USB or any other suitable interface.

The integral thermometer shown in Figs. 1 and 2 allows a user to determine a change in a baseline status or to predict an onset of an abnormal health condition of the examinee without the need for further equipment. However, the functionality of the thermometer according to the present invention may equally be realized by a thermometer having a sensor that is coupled to a remote computer that provides the required complementary processing functionality.

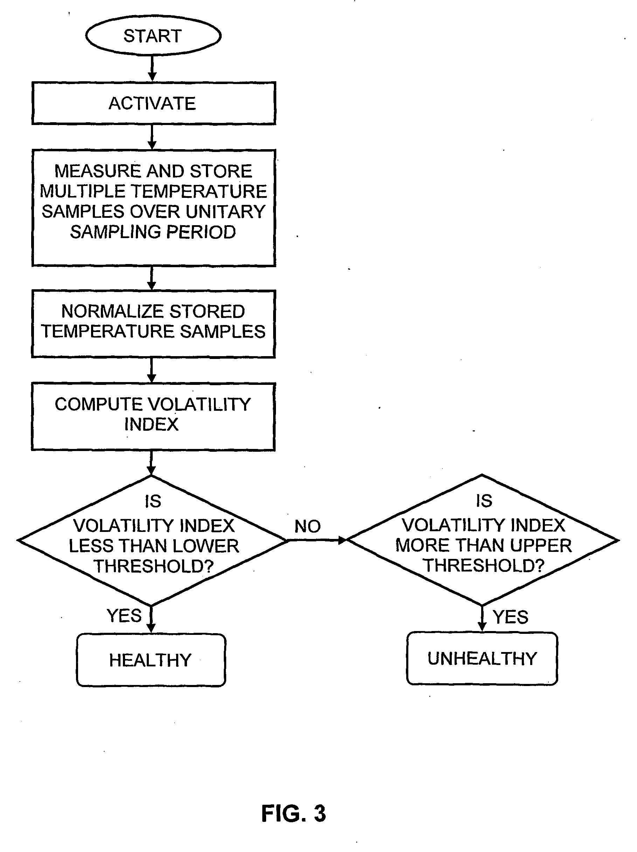

To understand how the thermometer 10 effects measurements, reference is now made to Fig. 3 showing the principal operations carried out by the processor 12 in accordance with an embodiment the invention. The thermometer 10 is activated by a start button, and is inserted into a patient's body cavity, such as the patient's mouth. Thereafter, under control of the clock 18, the processor 12 measures a plurality of temperature samples, each having a known time stamp, so that each temperature sample corresponds to a unique correlation between time and temperature. Once a sufficient number of measurements have been carried out, the results are normalized as explained below and a volatility index is computed. The volatility index allows determination as to whether the patient is healthy or not regardless of actual baseline temperature.

According to one embodiment the memory 14 may store a database of "patterns", i.e. known best-fit lines resulting from different health conditions such as sicknesses having characteristic temperature-time curves. A best-fit line that is "basically similar to" a stored pattern is indicative of an onset of health condition corresponding to the stored pattern.

Stored patterns may relate to any of the following:
**A viral disease such as influenza** - An examinee may sense the manifestation of a viral disease, although the symptoms thereof are not yet visible and the instantaneous body temperature is often less than that which would be considered by a physician as being characteristic of the viral disease. A best-fit line basically similar to the pattern of the viral disease is indicative of its manifestation, and therefore the thermometer of the invention assists a physician or patient in diagnosing the cause of discomfiture of the examinee and assists the physician in prescribing the proper steps to alleviate the discomfiture.
**Ovulation -** Females exhibit a higher body temperature of approximately one-quarter to one-half degree Celsius as progesterone is secreted after ovulation. Thus a best-fit line basically similar to a pattern of a woman who has initially entered the luteal phase, i.e. that part of the menstrual cycle that starts at ovulation and ends the day before the examinee's next period, is of great utility to those that are desirous of detecting periods of increased fertility by means of temperature measurement.
**Dehydration** - Hikers and sport practitioners frequently suffer from various levels of dehydration. Lack of moisture in the body, which is liable to lead to drowsiness and, at times, to the damage of bodily organs, may be avoided by detecting a best-fit line basically similar to the pattern of one that is suffering from dehydration.
**High blood pressure** - Some examinees exhibit a higher body temperature when their blood pressure increases. Thus a physician can utilize a best-fit line which is basically similar to the pattern of one suffering from heart disease or any other unusual health condition as a diagnostic tool.
**Hyperthermia, hypothermia, or a moderate fever** - An early manifestation of such an unusual health condition can be detected by the thermometer of the present invention before the instantaneous temperature reflects the health condition.

When using for diagnostic purposes, the measurements are generally administered by a physician to predict a change in a baseline status of the examinee and in particular to determine whether the examinee is undergoing an unusual health condition. The temperature probe may be inserted within any body cavity, such as the mouth, armpit or rectum. To allow the physician to review the data that is received and generated by the processor, including the stabilization time, the measuring time, the plurality of measurement data points, the best-fit line, the deviation of the best-fit line from a stored pattern, the straight line, the slope of the straight line, and the correlation value, the thermometer may also be provided with a Bluetooth™ or other suitable short range wireless transmitter. The measured and/or processed data is transmitted to a receiver of a suitable mobile device such as a cellular telephone or of a personal computer and may be stored for future reference. After the physician prescribes certain steps to be taken by the examinee, additional temperature measuring operations can be carried out as a follow-up by the physician, during which corresponding best-fit lines may be generated to determine whether the unusual health condition persists, is exacerbated, or recovery therefrom is predicted.

However, owing to the ease of use of the thermometer of the present invention, a temperature measuring operation can be self-administered by the examinee. After the examinee presses the start button and inserts the probe 13 into his body cavity, time-temperature data points are measured and normalized by the processor 12. If the processor 12 determines that the examinee is undergoing an unusual health condition, a textual message or an icon of the unusual health condition will be displayed. This display may also be accompanied by a suitable audible or visual alert.

To reduce the amount of memory stored within the pattern database and therefore the cost of the thermometer, each thermometer may be customized for a different sector. The patterns of those undergoing an unusual health condition may significantly vary from one sector to another. Exemplary sectors may include the following:
■ Males
■ Females
■ Pregnant women
■ The pediatric population
■ The geriatric population
■ Individuals suffering from heart conditions

### Examples

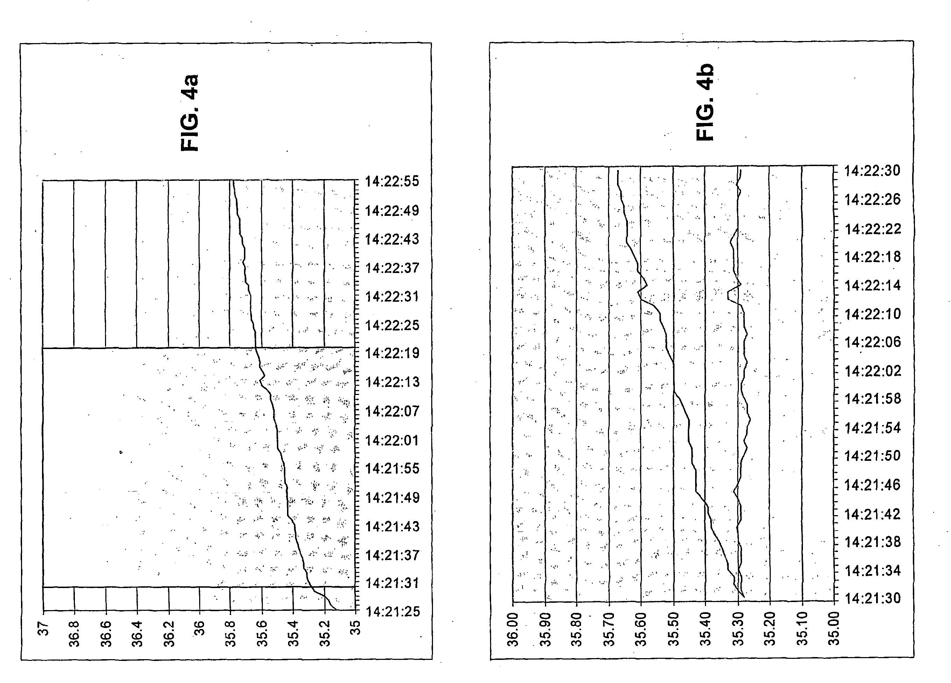

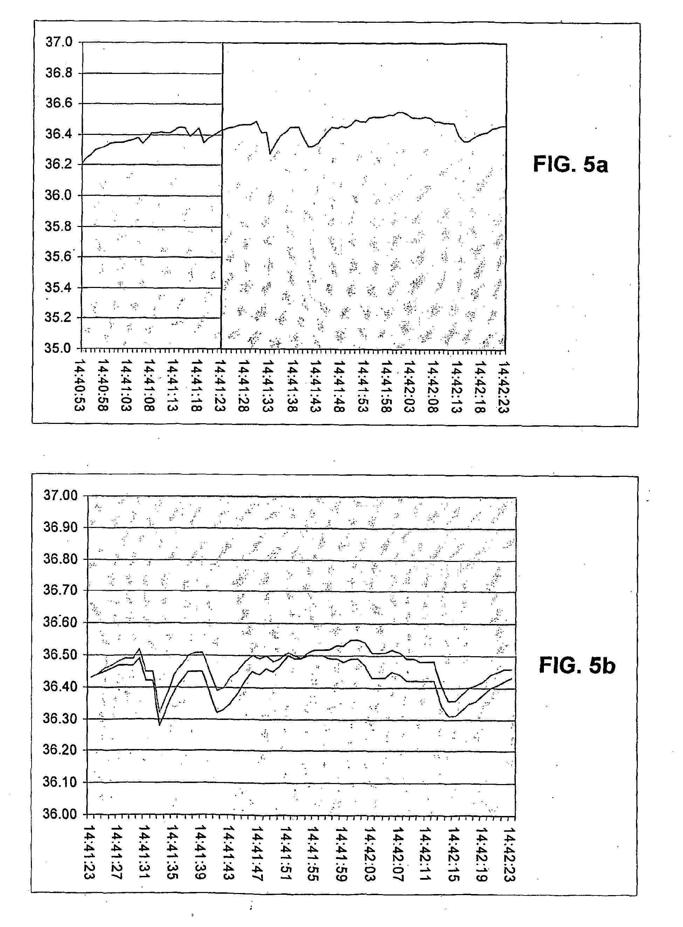

The temperatures of a series of healthy and non-healthy individuals were measured using a device according to the present invention and the results of the measurements will now be described with reference to Figs. 4 to 7, all of which show also the quantitative temperature values and associated time stamps.Figs. 4a and 5a are temperature-time characteristics of different healthy patients measured using the thermometer according to the invention, while Figs. 4b and 5b show the normalized temperature-time characteristics of Figs. 4a and 5a.

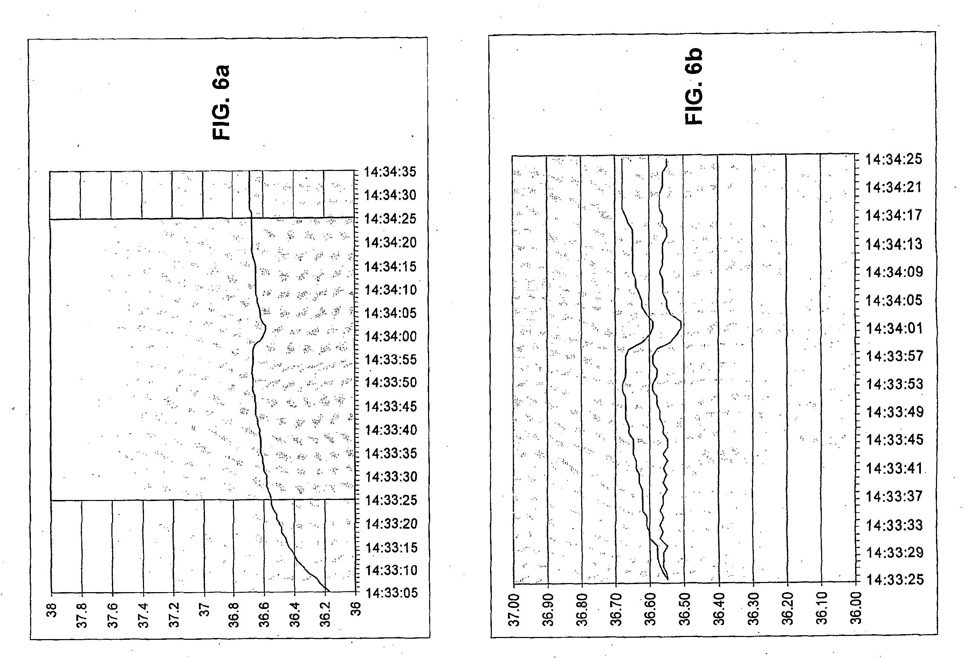

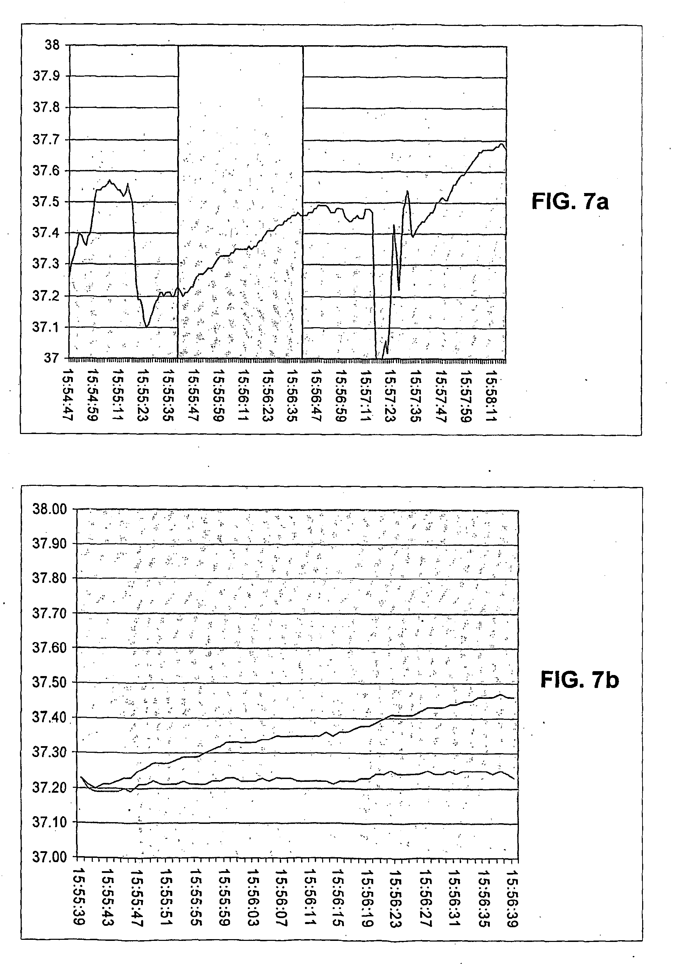

Figs. 6a and 7a are temperature-time characteristics of different unhealthy patients measured using the thermometer according to the invention, while Figs. 6b and 7b show the normalized temperature-time characteristics of Figs. 6a and 7a.

It is to be noted that a large number of different samples were taken and analyzed and for the sake of brevity only two samples from each population are shown. Although the measured temperature-time characteristics varied considerably, it was nevertheless found that all the processed measured temperature-time characteristics allow determination of whether the examinee is healthy or diseased to a statistically significant degree. Specifically, although the standard deviation of the samples of both healthy and unhealthy patients was almost identical, the median normalized volatility of the healthy patients was significantly lower than that of the unhealthy patients by more than two standard deviations. The normalized volatilities of healthy and unhealthy patients are tabulated in Tables I and 11, respectively, below from which it may be determined at least to a first approximation that a normalized volatility less than 29 is indicative of a healthy patient, while a normalized volatility greater than 32 is indicative of a sick patient.

**Table I**

| **ID #** | **Time** | **Cutoff from start** | **Normalized Volatility** |
|---|---|---|---|
| 044964 | 14:21:25 | Cutoff = 5 | 28.33 |
| 049818 | 14:37:56 | Cutoff = 30 | 31.67 |
| 331929 | 14:40:53 | Cutoff = 30 | 26.67 |
| 929044 | 14:48:23 | Cutoff = 30 | 31.67 |
| 453801 | 14:53:19 | Cutoff = 30 | 23.33 |
| 773918 | 14:56:20 | Cutoff = 30 | 30.00 |
| 540987 | 15:00:58 | Cutoff = 15 | 23.33 |
| 619371 | 15:45:54 | Cutoff = 30 | 28.33 |
| 929333 | 15:51:26 | Cutoff = 30 | 31.67 |
| 992372 | 16:02:36 | Cutoff = 30 | 28.33 |
| 978052 | 16:11:08 | Cutoff = 30 | 15.00 |
| 003301 | 11:27:06 | Cutoff = 15 | 20.00 |
| 003301 | 11:27:06 | Cutoff = 15 | 20.00 |
| 301109 | 12:21:06 | Cutoff = 20 | 21.67 |
| 605662 | 14:43:50 | Cutoff = 15 | 25.00 |
| 422033 | 14:48:17 | Cutoff = 15 | 10.00 |
| 243124 | 14:56:42 | Cutoff = 30 | 23.33 |
| 098762 | 14:59:44 | Cutoff = 30 | 25.00 |
| 929044 | 15:13:58 | Cutoff = 20 | 16.67 |
| 773918 | 15:47:14 | Cutoff = 20 | 28.33 |
| 049818 | 16:05:22 | Cutoff = 15 | 18.33 |
| 846095 | 16:12:56 | Cutoff = 20 | 13.33 |
| | | **Average** | **24.44** |
| | | **STD** | 5.68 |
| | | **Median** | 25.00 |

**Table II**

| **ID #** | **Time** | **Cutoff from start** | **Normalized Volatility** |
|---|---|---|---|
| 722801 | 14:33:05 | Cutoff = 20 | 35.00 |
| 610260 | 14:57:26 | Cutoff = 20 | 33.33 |
| 531019 | 15:54:47 | Cutoff = 52 | 36.67 |
| 531019 | 10:09:16 | Cutoff = 100 | 33.33 |
| 637445 | 12:23:00 | Cutoff = 12 | 30.00 |
| 531019 | 12:29:06 | Cutoff = 26 | 31.67 |
| 633678 | 12:34:49 | Cutoff = 21 | 36.67 |
| 425495 | 14:27:25 | Cutoff = 14 | 33.33 |
| 425495 | 14:11:10 | Cutoff = 10 | 38.33 |
| 531019 | 14:17:41 | Cutoff = 5 | 31.67 |
| 633678 | 14:38:31 | Cutoff = 30 | 35.00 |
| 425495 | 13:00:19 | Cutoff = 20 | 28.33 |
| 996079 | 09:13:56 | Cutoff = 15 | 38.33 |
| 973475 | 12:13:15 | Cutoff = 23 | 41.67 |
| 425495 | 12:22:38 | Cutoff = 15 | 33.33 |
| 425495 | 14:36:05 | Cutoff = 15 | 46.67 |
| 817668 | 14:14:45 | Cutoff = 8 | 30.00 |
| 386354 | 14:18:53 | Cutoff = 15 | 40.00 |
| 215710 | 14:28:46 | Cutoff = 20 | 48.33 |
| 215710 | 11:17:19 | Cutoff = 20 | 43.33 |
| 215710 | 10:35:48 | Cutoff = 30 | 30.00 |
| 547208 | 11:45:55 | Cutoff = 0 | 38.33 |
| | | **Average** | **36.06** |
| | | **STD** | 5.43 |
| | | **Median** | 35.00 |

Tables I and II will now be discussed with further reference to the sample temperature samples shown in Figs. 4 to 7. Observing data of fever dynamics taken over periods of 1.5-5min from a (growing) sample of both healthy and 'sick' people (i.e. someone with an infectious disease that does not have fever at the time of measuring), it was found that they differ in their volatility (i.e. the number of direction changes in the temperature graph during one measurement with the thermometer). This was obvious when the measured temperature was more or less stable with slight fluctuations during the measurement. However, most of the graphs showed a strange trend of being monotonically ascending from left to right. It is believed that this phenomenon suggesting that the temperature rises exactly when it is being measured probably results from warming of the mouth cavity when it is kept sealed for a minute or more. In any event, these unnatural monotonically increasing graphs have the unwanted effect of obscuring the true volatility. In order to overcome this, the graphs are normalized so as to de-skew the graphs whereby they are leveled while retaining their original topographic characteristics.

Once the graphs are normalized, the difference in the volatility between the two groups becomes readily apparent. For the small sample tabulated in Tables I and II, there is a difference of almost two standard deviations between the average volatility of the 'sick' and that of the healthy. Specifically, the average volatility for the healthy people is 24 while the median volatility for the 'sick' people is 36. The standard deviation (STD) for both groups is almost the same and small. This difference in median volatility is statistically significant, especially considering the small standard deviation of about 5.5. It is thus believed that volatility serves as a readily-measurable parameter that allows discrimination between healthy people and people who appear to be healthy in that they do not have fever but are probably not.

It will be appreciated that Table I includes data of some healthy patients whose normalized volatility is greater than 29 and Table II includes data of some healthy patients whose normalized volatility is less than 32. This suggests that there is a range of volatilities between 29 and 32 representing an area of uncertainty, as would be expected with any statistical analysis. But this does not contradict the overall finding that comparing normalized volatility with at least one threshold in most cases establishes whether the patient is healthy or sick.

Moreover, follow-ups taken over a period of several weeks have revealed that the invention may be used to predict a change in at least one health property of the examinee. Such prediction can serve to indicate that a person who is presently feeling well and does not exhibit a fever is nevertheless incubating a fever. The reverse has also been demonstrated whereby a patient with a chronic sickness was tested and found to have high volatility characteristic of sickness. The patient was given a prescription and told to return in two weeks for a further checkup. The patient was admitted as 'sick' but was found this time to have a low volatility indicating that the patient was on the road to recovery.

In another case, two patients were tested neither of whom had a fever and both of whom were feeling well. One was found to have low volatility while the other was found to have high volatility. It was predicted that the patient with high volatility was incubating a sickness and indeed within only 24 hours this was found to be the case.

In both tables, the columns labeled "Cutoff" show number of seconds skipped from the beginning of a measurement before a 60 second interval chosen for the volatility analysis. A "deskewing" algorithm was then applied to the original fever graphs for the chosen 60 seconds so as to eliminate the longer-term changes in the fever data. This has the effect of flattening the graphs while maintaining the all-important miniscule fluctuations.

The columns labeled "Normalized Volatility" show the number of direction changes along a graph divided by the graph's time interval (i.e. 60 sec in the sample results) multiplied by 100 resulting in a number between 0 and 100.

It will be evident to those skilled in the art that the invention is not limited to the details of the foregoing illustrative embodiments and that the present invention may be embodied in other specific forms without departing from the invention. The above-described embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

It will also be understood that a distributed thermometer according to the invention may utilize a suitably programmed computer. Likewise, the invention contemplates a computer program being readable by a computer for executing the method of the invention. The invention further contemplates a machine-readable memory tangibly embodying a program of instructions executable by the machine for executing the method of the invention.

Finally, it should be noted that the word "comprising" as used throughout the appended claims is to be interpreted to mean "including but not limited to".

## Claims

1. A digital thermometer (10), comprising a temperature measuring device (13) for measuring temperature of an examinee, and a microprocessor (12) coupled to the temperature measuring device for processing a sequence of temperature samples measured during a unitary measuring interval and being responsive to said temperature samples or a function thereof for determining at least one health property of the examinee or for predicting a change in at least one health property of the examinee;
**characterized in that:**
the temperature measuring device is adapted to measure to a resolution of at least 0.01°C at a sufficiently high sampling rate to yield measurable fluctuations between a plurality of sampled data points within the measuring interval and to enable a health property to be determined or a change in the health property predicted from said fluctuations even when each measured temperature sample exhibits substantially no departure from normal body temperature.

2. The thermometer according to claim 1, wherein the temperature measuring device is adapted to sample at a rate of 25 or more readings per minute.

3. The thermometer according to claim 1 or 2, wherein the measuring interval spans over a continuous period of time of up to several minutes.

4. The thermometer according to any one of claims 1 to 3, wherein the microprocessor is adapted to predict an onset of an abnormal health condition of the examinee or an onset of an abnormal temperature of the examinee.

5. The thermometer according to claim 4, wherein the microprocessor is adapted to compare said temperature samples or a function thereof with predetermined data characteristics of one or more abnormal health conditions for predicting an onset of an abnormal health condition of said examinee.

6. The thermometer according to any one of claims 1 to 5, wherein the microprocessor is programmed to reject readings that do not fulfill predetermined threshold criteria.

7. The thermometer according to claim 6, wherein a threshold criterion is any one in a group comprising (i) a maximum slope of a straight line generated between first and last data points of said best-fit line, (ii) a maximum deviation between two adjacent data points and (iii) a maximum deviation between one or more amplitudes of the best-fit line and the generated straight line.

8. The thermometer according to any one claims 1 to 7, further comprising a database of stored temperature fluctuation patterns each being characteristic of a respective health condition.

9. The thermometer according to claim 8, wherein the database is customized to store the patterns of a selected population sector.

10. The thermometer according to any one of claims 1 to 9, including a display unit (15) for displaying an icon or textual message indicative of a health condition of the examinee.

11. The thermometer according to any one of claims 1 to 10, including a vocalization unit (16) for vocalizing a tone or message.

12. The thermometer according to any one of claims 1 to 11, further comprising a communications unit (17) for conveying data that is received and generated by the microprocessor during the predetermined measuring time.

13. The thermometer according to claim 12, wherein said data is selected from the group of measuring time, stabilization time, a plurality of measured data points, the best-fit line, a deviation of the best-fit line from a stored pattern, the straight regression line, the slope of the straight line, and a maximum deviation between at least two adjacent data points.

14. The thermometer according to any one of claims 1 to 9, including a display device for displaying information indicative of a health condition of the examinee.

15. The thermometer according to any one of claims 1 to 14, wherein the microprocessor is adapted to compute a normalized volatility of the sequence of temperature samples and to compare the normalized volatility with a predetermined threshold for determining whether or not the examinee is healthy.

## Patentansprüche

1. Digitalthermometer (10) mit einer Temperaturmessvorrichtung (13) zum Messen der Temperatur einer zu untersuchenden Person und einem Mikroprozessor (12), der zum Verarbeiten einer Abfolge von Temperaturabfragewerten, die während eines einheitlichen Messintervalls gemessen werden, an die Temperaturmessvorrichtung angeschlossen ist und auf die Temperaturabfragewerte oder eine Funktion von ihnen anspricht, um mindestens ein Gesundheitsmerkmal der zu untersuchenden Person zu bestimmen oder eine Veränderung in mindestens einem Gesundheitsmerkmal der zu untersuchenden Person vorherzusagen,
**dadurch gekennzeichnet, dass**
die Temperaturmessvorrichtung geeignet ist, mit einer Auflösung von mindestens 0,01 °C bei einer ausreichend hohen Aufzeichnungsfrequenz zu messen, um messbare Schwankungen zwischen einer Vielzahl von abgefragten Datenpunkten innerhalb des Messintervalls zu erhalten und zu ermöglichen, dass ein Gesundheitsmerkmal bestimmt oder eine Veränderung mindestens eines Gesundheitsmerkmals aus den Schwankungen vorhergesagt wird, selbst wenn jeder gemessene Temperaturabfragewert im Wesentlichen keine Abweichung von der normalen Körpertemperatur zeigt.

2. Thermometer nach Anspruch 1, wobei die Temperaturmessvorrichtung zur Abfrage mit einer Frequenz von 25 oder mehr Ablesungen pro Minute geeignet ist.

3. Thermometer nach Anspruch 1 oder 2, wobei sich das Messintervall über einen zusammenhängenden Zeitraum von bis zu einigen Minuten erstreckt.

4. Thermometer nach einem der Ansprüche 1 bis 3, wobei der Mikroprozessor geeignet ist, ein Herausbilden eines abnormalen Gesundheitszustandes der zu untersuchenden Person oder ein Einsetzen einer abnormalen Temperatur der zu untersuchenden Person vorauszusagen.

5. Thermometer nach Anspruch 4, wobei der Mikroprozessor geeignet ist, die Temperaturabfragewerte oder eine Funktion von ihnen mit vorgegebenen Datenmerkmalen für einen oder mehrere abnormale Gesundheitszustände zu vergleichen, um ein Herausbilden eines abnormalen Gesundheitszustandes der zu untersuchenden Person vorherzusagen.

6. Thermometer nach einem der Ansprüche 1 bis 5, wobei der Mikroprozessor programmiert ist, Ablesungen zurückzuweisen, die vorgegebene Schwellenwertkriterien nicht erfüllen.

7. Thermometer nach Anspruch 6, wobei ein Schwellenwertkriterium ein beliebiges aus einer Gruppe ist, die umfasst: (i) einen maximalen Anstieg einer geraden Linie, die zwischen den ersten und den letzten Datenpunkten der Ausgleichsgeraden erzeugt wird; (ii) eine maximale Abweichung zwischen zwei benachbarten Datenpunkten; und (iii) eine maximale Abweichung zwischen einer oder mehreren Amplituden der Ausgleichsgeraden und der erzeugten geraden Linie.

8. Thermometer nach einem der Ansprüche 1 bis 7, ferner eine Datenbank der gespeicherten Temperaturschwankungsmuster umfassend, von denen jedes charakteristisch für einen jeweiligen Gesundheitszustand ist.

9. Thermometer nach Anspruch 8, wobei die Datenbank anwenderspezifisch ist, um die Muster eines ausgewählten Bevölkerungsanteils zu speichern.

10. Thermometer nach einem der Ansprüche 1 bis 9, das eine Anzeigeeinheit (15) enthält, um ein Bildzeichen oder eine Textnachricht anzuzeigen, die einen Hinweis auf den Gesundheitszustand der zu überprüfenden Person gibt.

11. Thermometer nach einem der Ansprüche 1 bis 10, das eine akustische Ausgabeeinheit (16) enthält, um die akustische Ausgabe eines Tones oder einer Nachricht zu bewirken.

12. Thermometer nach einem der Ansprüche 1 bis 11, das ferner eine Kommunikationseinheit (17) umfasst, um die Daten zu übermitteln, die während der vorgegebenen Messzeit empfangen und durch den Mikroprozessor erzeugt wurden.

13. Thermometer nach Anspruch 12, wobei die Daten ausgewählt sind aus der Gruppe aus Messzeit, Stabilisierungszeit, mehreren gemessenen Datenpunkten, Ausgleichsgerade, einer Abweichung der Ausgleichsgeraden von einem gespeicherten Muster, Regressionsgerade, Anstieg der Geraden und einer maximalen Abweichung zwischen mindestens zwei benachbarten Datenpunkten.

14. Thermometer nach einem der Ansprüche 1 bis 9, das eine Anzeigevorrichtung für die Anzeige von Informationen enthält, die einen Hinweis auf den Gesundheitszustand der zu überprüfenden Person geben.

15. Thermometer nach einem der Ansprüche 1 bis 14, wobei der Mikroprozessor geeignet ist, eine normierte Volatilität der Abfolge von Temperaturabfragewerten zu berechnen und die normierte Volatilität mit einem vorgegebenen Schwellenwert zu vergleichen, um zu bestimmen, ob die zu untersuchende Person gesund ist oder nicht.

## Revendications

1. Thermomètre numérique (10), comprenant un dispositif de mesure de température (13) pour mesurer la température d'une personne examinée, et un microprocesseur (12) couplé au dispositif de mesure de température pour traiter une séquence d'échantillons de température mesurés au cours d'un intervalle de mesure unitaire et étant réactif aux dits échantillons de température ou à une fonction de celui-ci pour déterminer au moins une propriété de santé de la personne examinée ou pour prédire un changement d'au moins une propriété de santé de la personne examinée ;
**caractérisé en ce que:**
le dispositif de mesure de température est apte à mesurer à une résolution d'au moins 0,01°C à une fréquence d'échantillonnage suffisamment haute pour donner des fluctuations mesurables entre une pluralité de points de données échantillonnés dans l'intervalle de mesure et pour permettre de déterminer une propriété de santé ou de prédire un changement de la propriété de santé à partir desdites fluctuations même lorsque chaque échantillon de température mesuré ne présente sensiblement aucun écart par rapport à la température corporelle normale.

2. Thermomètre selon la revendication 1, dans lequel le dispositif de mesure de température est apte à échantillonner à une fréquence d'au moins 25 relevés par minute.

3. Thermomètre selon la revendication 1 ou 2, dans lequel l'intervalle de mesure couvre une période continue de temps jusqu'à plusieurs minutes.

4. Thermomètre selon l'une quelconque des revendications 1 à 3, dans lequel le microprocesseur est apte à prédire le commencement d'un état de santé anormal de la personne examinée ou le commencement d'une température anormale de la personne examinée.

5. Thermomètre selon la revendication 4, dans lequel le microprocesseur est apte à comparer lesdits échantillons de température ou une fonction de celui-ci à des caractéristiques de données prédéterminées d'un ou plusieurs états de santé anormaux pour prédire le commencement d'un état de santé anormal de ladite personne examinée.

6. Thermomètre selon l'une quelconque des revendications 1 à 5, dans lequel le microprocesseur est programmé pour rejeter les relevés qui ne répondent pas à des critères de seuil prédéterminés.

7. Thermomètre selon la revendication 6, dans lequel un critère de seuil est l'un quelconque d'un groupe comprenant (i) une pente maximale d'une ligne droite générée entre les premier et dernier points de données et de ladite ligne de meilleure correspondance, (ii) un écart maximal entre deux points de données adjacents et (iii) un écart maximal entre une ou plusieurs amplitudes de la ligne de meilleure correspondance et la ligne droite générée.

8. Thermomètre selon l'une quelconque des revendications 1 à 7, comprenant en outre une base de données de motifs de fluctuations de température stockés qui sont chacun caractéristiques d'un état de santé respectif.

9. Thermomètre selon la revendication 8, dans lequel la base de données est personnalisée pour stocker les motifs d'un secteur de population sélectionné.

10. Thermomètre selon l'une quelconque des revendications 1 à 9, comprenant une unité d'affichage (15) pour afficher une icône ou un message textuel indiquant un état de santé de la personne examinée.

11. Thermomètre selon l'une quelconque des revendications 1 à 10, comprenant une unité de vocalisation (16) pour vocaliser un son ou un message.

12. Thermomètre selon l'une quelconque des revendications 1 à 11, comprenant en outre une unité de communication (17) pour transporter les données qui sont reçues et générées par le microprocesseur pendant le temps de mesure prédéterminé.

13. Thermomètre selon la revendication 12, dans lequel lesdites données sont sélectionnées dans le groupe se composant d'un temps de mesure, un temps de stabilisation, une pluralité de points de données mesurés, la ligne de meilleure correspondance, un écart de la ligne de meilleure correspondance par rapport à un motif stocké, la ligne de régression droite, la pente de la ligne droite, et un écart maximum entre au moins deux points de données adjacents.

14. Thermomètre selon l'une quelconque des revendications 1 à 9, comprenant un dispositif d'affichage pour afficher des informations indiquant un état de santé de la personne examinée.

15. Thermomètre selon l'une quelconque des revendications 1 à 14, dans lequel le microprocesseur est apte à calculer une volatilité normalisée de la séquence d'échantillons de température et à comparer la volatilité normalisée à un seuil prédéterminé pour déterminer si la personne examinée est en bonne santé.
